# EUROPEAN PATENT APPLICATION

(11) **EP 2 012 127 A2**
(43) Date of publication of application: **07.01.2009**
(21) Application number: 07111598.4
(22) Date of filing: 03.07.2007
(51) Int. Cl.: G01N 33/574

(54) **Diagnostic markers for detecting prostate cancer**

(71) Applicant: Koninklijke Philips Electronics N.V., 5621 BA Eindhoven (NL); Philips Intellectual Property & Standards GmbH, 20099 Hamburg (DE)
(72) Inventor: The designation of the inventor has not yet been filed
(74) Representative: Plünnecke, Ingo

(57) **Abstract**

The present invention is concerned with a set of marker molecules for diagnosing prostate cancer.

## Description

### SUBJECT OF THE INVENTION

The invention relates the field of detecting prostate cancer. The invention is concerned *inter alia* with diagnostic marker molecules for detecting prostate cancer, with the use of such molecules for detecting prostate cancer and with methods of diagnosing prostate cancer.

### BACKGROUND OF THE INVENTION

Diagnosis of diseases often relies on a variety of parameters in the absence of an available disease-specific molecular marker.

For example, prostate cancer is the most common male malignancy and the second leading cause of male cancer-related death in the United States. It accounts for 29% of all male cancers and 11 % of male cancer-related death. Small prostate carcinomas were detected in 30% of men aged 30 to 49 years, in 40% of males over age 50 and in 64 % of men aged 60 to 70 years (Sakr et. al. (1993) J. Urol., 150:379-385).

In view of the incidence of prostate cancer, a reliable diagnostic method that is capable of predicting this disease at an early stage with high reliability is obviously of major importance.

Currently, prostate cancer screening is typically based on a combination of three diagnostic approaches, namely physical examination, biochemical examination and image analysis. A diagnostic marker for making an ultimate decision whether prostate cancer development is ongoing or not is not yet available.

Physical examination is typically performed by digital rectal examination (DRE). Other approaches include examination of the abdomen by inspection, percussion and palpation in order to detect any distension, palpable renal masses and enlargement of the bladder or tenderness.

Biochemical analysis, to a large extent, relies on monitoring serum for the level of prostate-specific antigen (PSA). The normally accepted upper level for serum PSA which is determined by a monoclonal antibody in an immunoassay is 4 ng/ml. However, some men with prostate cancer may have normal levels of PSA and elevated levels of PSA may also be due to other factors, such as age, infarction etc. Thus, PSA monitoring as well as analysis of other biochemical factors is in itself insufficient to either exclude or confirm the presence of a tumor (Thompson et al. (2005) JAMA, 294, 66-70).

As a consequence, the two aforementioned diagnostic approaches, namely DRE and PSA monitoring are usually supported by trans-rectal ultrasound (TRUS) guided biopsy analysis.

To this end, the biopsy is usually targeted to a focal area of abnormal echogenicity or to an area of palpable abnormality. As the area of tumor development may not easily be identifiable in the trans-rectal ultrasound analysis, one will take six, twelve or sometimes more biopsies from different areas of the prostate during a normal ultrasound scan. These targeted biopsies are then often combined with systematic biopsies in an individual patient which is followed by subsequent histological analysis.

Even though histological analysis, if performed by an experienced histopathologist, may usually give a good indication of whether a tumor is malignant or benign, the number of false-negative diagnosis is considerable. Thus, each biopsy may miss the presence of prostate cancer including even the most aggressive forms. TRUS-guided needle biopsy can miss up to 34% of clinically localized prostate cancers and about 10 to 19% of patients with initially negative needle biopsy were diagnosed with prostate on a second biopsy (Keetch et. al. (1994), J. Urol., 151:1571-1574). Taken together, none of the aforementioned currently practiced standard methods are sufficiently sensitive and specific for reliably diagnosing prostate cancer which makes early detection of this cancer difficult.

There is thus a continuing need for diagnostic markers and methods of diagnosis which allow for reliable detection of prostate cancer in human subjects.

### OBJECT AND SUMMARY OF THE INVENTION

It is one objective of the present invention to provide diagnostic markers which allow for specific and reliable detection of prostate cancer in human subjects.

It is another objective of the present invention to provide for methods of diagnosis that allow reliable and specific detection of prostate cancer in human subjects.

These and other objectives of the present invention, as they will become apparent from the ensuing description are solved by the subject matter of the independent claims. The dependent claims relate to some of the preferred embodiments of the present invention.

The present invention is based on the identification of different diagnostic marker molecules that are derived from proteins, the expression of which is specifically up- or down-regulated in cells of human subjects being afflicted by ongoing prostate cancer development.

The present invention in one of its embodiments thus relates to a diagnostic marker for detecting prostate cancer comprising at least one marker molecule selected from the group consisting of:
Marker molecule 1 having a molecular weight of about 8092 Da;
Marker molecule 2 having a molecular weight of about 8296 Da;
Marker molecule 3 having a molecular weight of about 10104 Da;
Marker molecule 4 having a molecular weight of about 10511 Da;
Marker molecule 5 having a molecular weight of about 13383 Da;
Marker molecule 6 having a molecular weight of about 14998 Da;
Marker molecule 7 having a molecular weight of about 15193 Da;
Marker molecule 8 having a molecular weight of about 15481 Da;
Marker molecule 9 having a molecular weight of about 15755 Da;
Marker molecule 10 having a molecular weight of about 16714 Da;
Marker molecule 11 having a molecular weight of about 16915 Da;
Marker molecule 12 having a molecular weight of about 19851 Da;
Marker molecule 13 having a molecular weight of about 22209 Da;
Marker molecule 14 having a molecular weight of about 22719 Da;
Marker molecule 15 having a molecular weight of about 32741 Da;
Marker molecule 16 having a molecular weight of about 33622 Da;
Marker molecule 17 having a molecular weight of about 2971 Da;
Marker molecule 18 having a molecular weight of about 4018 Da;
Marker molecule 19 having a molecular weight of about 7524 Da;
Marker molecule 20 having a molecular weight of about 8479 Da;
Marker molecule 21 having a molecular weight of about 12176 Da;
Marker molecule 22 having a molecular weight of about 13497 Da;
Marker molecule 23 having a molecular weight of about 15326 Da;
Marker molecule 24 having a molecular weight of about 16469 Da;
Marker molecule 25 having a molecular weight of about 16783 Da;
Marker molecule 26 having a molecular weight of about 16912 Da;
Marker molecule 27 having a molecular weight of about 17163 Da;
Marker molecule 28 having a molecular weight of about 19795 Da; and
Marker molecule 29 having a molecular weight of about 22189 Da.

Such a diagnostic marker molecule may preferably be Marker molecule 11 having a molecular weight of about 16915 Da; or Marker molecule 26 having a molecular weight of about 16912 Da.

In a preferred embodiment the diagnostic marker comprises a group of at least 4 marker molecule, a group of at least 8 marker molecules, a group of at least 12 marker molecules and, in a further preferred embodiment a group of 16 marker molecules with the marker molecules being selected from the aforementioned Marker molecules 1 to 16.

These groups may preferably comprise at least the four marker molecules:
Marker molecule 11 having a molecular weight of about 16915 Da;
Marker molecule 10 having a molecular weight of about 16714 Da;
Marker molecule 1 having a molecular weight of about 8092 Da; and
Marker molecule 14 having a molecular weight of about 22719 Da.

In another preferred embodiment the diagnostic marker comprises a group of at least 4 marker molecule, a group of at least 8 marker molecules, a group of at least 12 marker molecules and, in a further preferred embodiment a group of 13 marker molecules with the marker molecules being selected from the Marker molecules 17 to 29.

These groups may preferably comprise at least the four marker molecules:
Marker molecule 26 having a molecular weight of about 16912 Da;
Marker molecule 25 having a molecular weight of about 16783 Da;
Marker molecule 27 having a molecular weight of about 17163 Da; and
Marker molecule 20 having a molecular weight of about 8479 Da.

The diagnostic marker molecules are obtainable by analyzing a sample from a subject which is suspected to be afflicted by prostate cancer, relying on mass spectrometry, including matrix-assisted laser desorption ionization mass spectrometry, electron spray ionization mass spectrometry, surface enhanced laser desorption ionization mass spectrometry and other mass spectrometry technologies.

In another embodiment the present invention relates to the use of at least one marker molecule selected from the group consisting of:
Marker molecule 1 having a molecular weight of about 8092 Da;
Marker molecule 2 having a molecular weight of about 8296 Da;
Marker molecule 3 having a molecular weight of about 10103 Da;
Marker molecule 4 having a molecular weight of about 10511 Da;
Marker molecule 5 having a molecular weight of about 13383 Da;
Marker molecule 6 having a molecular weight of about 14998 Da;
Marker molecule 7 having a molecular weight of about 15193 Da;
Marker molecule 8 having a molecular weight of about 15481 Da;
Marker molecule 9 having a molecular weight of about 15755 Da;
Marker molecule 10 having a molecular weight of about 16714 Da;
Marker molecule 11 having a molecular weight of about 16915 Da;
Marker molecule 12 having a molecular weight of about 19851 Da;
Marker molecule 13 having a molecular weight of about 22209 Da;
Marker molecule 14 having a molecular weight of about 22719 Da;
Marker molecule 15 having a molecular weight of about 32741 Da;
Marker molecule 16 having a molecular weight of about 33622 Da;
Marker molecule 17 having a molecular weight of about 2971 Da;
Marker molecule 18 having a molecular weight of about 4018 Da;
Marker molecule 19 having a molecular weight of about 7524 Da;
Marker molecule 20 having a molecular weight of about 8479 Da;
Marker molecule 21 having a molecular weight of about 12176 Da;
Marker molecule 22 having a molecular weight of about 13497 Da;
Marker molecule 23 having a molecular weight of about 15326 Da;
Marker molecule 24 having a molecular weight of about 16469 Da;
Marker molecule 25 having a molecular weight of about 16783 Da;
Marker molecule 26 having a molecular weight of about 16912 Da;
Marker molecule 27 having a molecular weight of about 17163 Da;
Marker molecule 28 having a molecular weight of about 19795 Da; and
Marker molecule 29 having a molecular weight of about 22189 Da.

The at least one marker molecule which is used in the diagnosis of prostate cancer can be preferably:
Marker molecule 11 having a molecular weight of about 16915 Da; or
Marker molecule 26 having a molecular weight of about 16912 Da.

In a preferred embodiment, a group of at least 4 marker molecules, of at least 8 marker molecules, of at least 12 marker molecules and in a further preferred embodiment of 16 marker molecules is used for diagnosing prostate cancer in a human subject with the marker molecules of these groups being selected from the aforementioned Marker molecules 1 to 16.

In a preferred embodiment these groups may comprise at least:
Marker molecule 11 having a molecular weight of about 16915 Da;
Marker molecule 10 having a molecular weight of about 16714 Da;
Marker molecule 1 having a molecular weight of about 8092 Da; and
Marker molecule 14 having a molecular weight of about 22719 Da.

In another preferred embodiment, a group of at least 4 marker molecules, of at least 8 marker molecules, of at least 12 marker molecules and in a further preferred embodiment of 13 marker molecules is used for diagnosing prostate cancer in a human subject with the marker molecules of these groups being selected from the aforementioned Marker molecules 17 to 29.

In a preferred embodiment these groups may comprise at least:
Marker molecule 26 having a molecular weight of about 16912 Da;
Marker molecule 25 having a molecular weight of about 16783 Da;
Marker molecule 27 having a molecular weight of about 17163 Da; and
Marker molecule 20 having a molecular weight of about 8479 Da.

The present invention is also concerned with a method of aiding a prostate cancer diagnosis in a subject comprising the step of:
a) detecting at least one marker molecule in a sample from said subject wherein
   the marker molecule is selected from the group consisting of:
   Marker molecule 1 having a molecular weight of about 8092 Da;
   Marker molecule 2 having a molecular weight of about 8296 Da;
   Marker molecule 3 having a molecular weight of about 10104 Da;
   Marker molecule 4 having a molecular weight of about 10511 Da;
   Marker molecule 5 having a molecular weight of about 13383 Da;
   Marker molecule 6 having a molecular weight of about 14998 Da;
   Marker molecule 7 having a molecular weight of about 15193 Da;
   Marker molecule 8 having a molecular weight of about 15481 Da;
   Marker molecule 9 having a molecular weight of about 15755 Da;
   Marker molecule 10 having a molecular weight of about 16714 Da;
   Marker molecule 11 having a molecular weight of about 16915 Da;
   Marker molecule 12 having a molecular weight of about 19851 Da;
   Marker molecule 13 having a molecular weight of about 22209 Da;
   Marker molecule 14 having a molecular weight of about 22719 Da;
   Marker molecule 15 having a molecular weight of about 32741 Da;
   Marker molecule 16 having a molecular weight of about 33622 Da;
   Marker molecule 17 having a molecular weight of about 2971 Da;
   Marker molecule 18 having a molecular weight of about 4018 Da;
   Marker molecule 19 having a molecular weight of about 7524 Da;
   Marker molecule 20 having a molecular weight of about 8479 Da;
   Marker molecule 21 having a molecular weight of about 12176 Da;
   Marker molecule 22 having a molecular weight of about 13497 Da;
   Marker molecule 23 having a molecular weight of about 15326 Da;
   Marker molecule 24 having a molecular weight of about 16469 Da;
   Marker molecule 25 having a molecular weight of about 16783 Da;
   Marker molecule 26 having a molecular weight of about 16912 Da;
   Marker molecule 27 having a molecular weight of about 17163 Da;
   Marker molecule 28 having a molecular weight of about 19795 Da; and
   Marker molecule 29 having a molecular weight of about 22189 Da.

In another embodiment, the present invention is concerned with a method for diagnosing prostate cancer in a subject comprising the steps of:
a) detecting at least one marker molecule in a sample from said subject wherein
   the marker molecule is selected from the group consisting of:
   Marker molecule 1 having a molecular weight of about 8092 Da;
   Marker molecule 2 having a molecular weight of about 8296 Da;
   Marker molecule 3 having a molecular weight of about 10104 Da;
   Marker molecule 4 having a molecular weight of about 10511 Da;
   Marker molecule 5 having a molecular weight of about 13383 Da;
   Marker molecule 6 having a molecular weight of about 14998 Da;
   Marker molecule 7 having a molecular weight of about 15193 Da;
   Marker molecule 8 having a molecular weight of about 15481 Da;
   Marker molecule 9 having a molecular weight of about 15755 Da;
   Marker molecule 10 having a molecular weight of about 16714 Da;
   Marker molecule 11 having a molecular weight of about 16915 Da;
   Marker molecule 12 having a molecular weight of about 19851 Da;
   Marker molecule 13 having a molecular weight of about 22209 Da;
   Marker molecule 14 having a molecular weight of about 22719 Da;
   Marker molecule 15 having a molecular weight of about 32741 Da;
   Marker molecule 16 having a molecular weight of about 33622 Da;
   Marker molecule 17 having a molecular weight of about 2971 Da;
   Marker molecule 18 having a molecular weight of about 4018 Da;
   Marker molecule 19 having a molecular weight of about 7524 Da;
   Marker molecule 20 having a molecular weight of about 8479 Da;
   Marker molecule 21 having a molecular weight of about 12176 Da;
   Marker molecule 22 having a molecular weight of about 13497 Da;
   Marker molecule 23 having a molecular weight of about 15326 Da;
   Marker molecule 24 having a molecular weight of about 16469 Da;
   Marker molecule 25 having a molecular weight of about 16783 Da;
   Marker molecule 26 having a molecular weight of about 16912 Da;
   Marker molecule 27 having a molecular weight of about 17163 Da;
   Marker molecule 28 having a molecular weight of about 19795 Da; and
   Marker molecule 29 having a molecular weight of about 22189 Da.
b) comparing the amount of said marker with the amount of the same marker molecule in a reference sample from a subject which is known to not be affected by prostate cancer,
c) diagnosing prostate cancer if said at least one marker molecule is found to be differentially expressed compared to said standard sample.

The aforementioned methods of aiding a prostate cancer diagnosis or diagnosing prostate cancer in a human subject preferably rely on at least one marker being selected from:
Marker molecule 11 having a molecular weight of about 16915 Da; or
Marker molecule 26 having a molecular weight of about 16912 Da.

The aforementioned methods may also preferably be performed with a group of at least 4 marker molecules, a group of at least 8 marker molecules, a group of at least 12 marker molecules and particularly preferably a group of 16 marker molecules with the marker molecules being selected from the aforementioned Marker molecules 1 to Marker molecule 16. These groups may comprise at least
Marker molecule 11 having a molecular weight of about 16915 Da;
Marker molecule 10 having a molecular weight of about 16714 Da;
Marker molecule 1 having a molecular weight of about 8092 Da; and
Marker molecule 14 having a molecular weight of about 22719 Da.

In such a preferred embodiment the diagnosis may be made with a likelihood of at least about 75%.

The aforementioned methods may also preferably be performed with a group of at least 4 marker molecules, a group of at least 8 marker molecules, a group of at least 12 marker molecules and particularly preferably a group of 13 marker molecules with the marker molecules being selected from the aforementioned Marker molecules 17 to 29. These groups may comprise at least
Marker molecule 26 having a molecular weight of about 16912 Da;
Marker molecule 25 having a molecular weight of about 16783 Da;
Marker molecule 27 having a molecular weight of about 17163 Da; and
Marker molecule 20 having a molecular weight of about 8479 Da.

In such a preferred embodiment the diagnosis may be made with a likelihood of at least about 75%.

For the method of diagnosis it can be preferred to analyze a sample which is not in direct contact with the human or animal body. Thus, the method of diagnosis does not require direct interaction with the human or animal body. Such samples may, e.g. be a full blood sample, a serum sample, a tissue sample, a saliva sample all of which are obtained from a subject being suspected of suffering from prostate cancer.

The samples may be taken from subjects that are suspected to suffer from prostate cancer development. One may also rely on the markers and marker combinations as mentioned herein to analyze biopsy samples from such subjects which show histological abnormalities.

### DETAILED DESCRIPTION OF THE INVENTION

Surprisingly it has been found that a certain set of marker molecules can be used for aiding a diagnosis of prostate cancer due to differential expression of these markers in samples derived from a subject afflicted by prostate cancer versus a subject which is not afflicted by prostate cancer.

Before some of the embodiments of the present invention are described in more detail, the following definitions are introduced.

As used in the specification and in the claims, the singular forms of "a" and "an" also include the respective plurals unless the context clearly dictates otherwise.

The terms "about" and "approximately" in the context of the present invention generally denote a level or interval accuracy that a person skilled in the art will understand to still ensure the technical effect of the feature in question. As regards numerical values, these terms typically indicate a deviation from the indicated numerical value of ± 10% and preferably of ± 5%.

If the terms "about" and "approximately" are used in conjunction with the numerical value of a molecular weight of a marker molecule, the terms indicate a deviation from the indicated molecular weight at maximum of ± 0.5 %, preferably at maximum of ± 0.3 %, more preferably at maximum ± 0.2 % and most preferably at maximum ± 0.1 %.

It is to be understood that the term "comprising" is not limiting. For the purposes of the present invention, the term "consisting of" is considered to be a preferred embodiment of the term "comprising of". If hereinafter a group is defined to comprise at least a certain number of embodiments, this is also meant to disclose a group which preferably consists of these embodiments only.

Further definitions of term will be given in the context of which the terms are used.

Cancer development is typically characterised by an aberrant regulation of molecular signal pathways within cells. Aberrant regulation of molecular signalling may lead to a situation where a certain cellular factor such as a protein is expressed only in cancer tissue, but not in non-cancerogenic tissue. Such proteins may e.g. hybrid proteins resulting from chromosomal translocations of gene fragments and thus represent molecular entities which are not at all present in non-cancerogenic tissues. In other types of cancer, such proteins may be characterised in that they carry e.g. a specific point mutation which is not found in normal, non-cancerogenic tissue.

However, in a lot of cancer types, aberrant regulation of molecular signalling pathways does not lead to the occurrence of a completely new cellular factor, but to the alteration of the amounts of cellular factors. In other words, a cancer may be characterised by the up-regulated or down-regulated expression of certain types of proteins which are also present in non-cancerogenic tissue albeit at different levels. In principle, it should therefore be possible to diagnose a cancer type on the basis of an altered expression of cellular factors such as proteins which are either up- or down-regulated during cancer development in comparison to normal, non-cancerogenic tissue. However, as is known to the person skilled in the art, an altered expression of a protein may not necessarily be indicative of ongoing cancer development but also may result from normal intra-individual differences between healthy human subjects.

The inventors of the present invention have succeeded in identifying a set of marker molecules which can be used to reliably diagnose prostate cancer in human subjects as a consequence of their differential expression in cancerogenic versus non-cancerogenic tissues. An alteration in the amount of these molecular markers will be indicative of prostate cancer even if the aforementioned intra individual differences in protein expression in normal non-cancerogenic tissue are taken into account.

Thus, the present invention relates in one embodiment to a diagnostic marker for detecting prostate cancer comprising at least one marker molecule selected from the group consisting of:
Marker molecule 1 having a molecular weight of about 8092 Da;
Marker molecule 2 having a molecular weight of about 8297 Da;
Marker molecule 3 having a molecular weight of about 10104 Da;
Marker molecule 4 having a molecular weight of about 10511 Da;
Marker molecule 5 having a molecular weight of about 13383 Da;
Marker molecule 6 having a molecular weight of about 14998 Da;
Marker molecule 7 having a molecular weight of about 15193 Da;
Marker molecule 8 having a molecular weight of about 15481 Da;
Marker molecule 9 having a molecular weight of about 15755 Da;
Marker molecule 10 having a molecular weight of about 16714 Da;
Marker molecule 11 having a molecular weight of about 16915 Da;
Marker molecule 12 having a molecular weight of about 19851 Da;
Marker molecule 13 having a molecular weight of about 22209 Da;
Marker molecule 14 having a molecular weight of about 22719 Da;
Marker molecule 15 having a molecular weight of about 32741 Da;
Marker molecule 16 having a molecular weight of about 33622 Da;
Marker molecule 17 having a molecular weight of about 2971 Da;
Marker molecule 18 having a molecular weight of about 4018 Da;
Marker molecule 19 having a molecular weight of about 7524 Da;
Marker molecule 20 having a molecular weight of about 8479 Da;
Marker molecule 21 having a molecular weight of about 12176 Da;
Marker molecule 22 having a molecular weight of about 13497 Da;
Marker molecule 23 having a molecular weight of about 15326 Da;
Marker molecule 24 having a molecular weight of about 16469 Da;
Marker molecule 25 having a molecular weight of about 16783 Da;
Marker molecule 26 having a molecular weight of about 16912 Da;
Marker molecule 27 having a molecular weight of about 17163 Da;
Marker molecule 28 having a molecular weight of about 19795 Da; and
Marker molecule 29 having a molecular weight of about 22189 Da.

In the following it will be discussed how these marker molecules can be used for prostate cancer diagnosis in a human subject. Further specifics of the markers as well as the methods of diagnosis and methods of aiding prostate cancer diagnosis will be discussed further below.

Marker molecules of the above mentioned group of Marker molecule 1 to Marker molecule 29 can be used alone or in combination for detecting prostate cancer in a human subject due to their differential expression in cancerogenic versus non-cancerogenic tissue.

In the context of diagnosis, one will typically obtain e.g. a tissue sample from a human subject which is suspected to suffer from prostate cancer. Subsequently, this sample may be analysed by mass spectrometry approaches as discussed below.

Following this analysis one will observe a protein expression pattern (also called profile) that is attributable to the sample and will compare this protein expression profile with the expression profile of a reference sample that has been derived from a human subject that is known not to suffer from prostate cancer. In a subsequent step one then determines and how many of the marker molecules of Marker molecules 1 to 29 are present in the sample of the subject suspected to suffer from prostate cancer development and the reference sample. Subsequently the amount of the marker molecules identified are compared and if a significant differential expression is observed, a diagnosis as to ongoing prostate cancer development can be made.

As used herein, "diseases of the prostate" refer to any disease or condition of the prostate including but not limited to benign prostatic hyperplasia (BPH), prostatitis, prostatic intraepithelial neoplasia (PIN) and cancer.

As used herein "prostate cancer" refers to any malignant disease of the prostate including but not limited to adenocarcinoma, small cell undifferentiated carcinoma and mucinous (colloid) cancer.

As used herein, the term "metastatic prostate cancer" relates to prostate cancers which have spread to regional lymph nodes or to distant sites. As used herein, the terms "locally advanced prostate cancer" mean prostate cancers which have extended through the prostate capsule. The systems that are used in the art for classifying prostate cancers may also be considered in this context. These systems include the American Urological Association (AUA) system, the WHITMORE-JEWETT system and the Tumour Node Metastasis System (TNM).

As used herein, the term "subject" encompasses any human male subject that is suspected to suffer from prostate cancer and/or which is suspected to be afflicted by ongoing prostate cancer development.

The term "sample" refers to any sample which can be taken from a human male subject in order to perform a test for the presence of the marker molecules as described herein. A sample will typically be a whole blood sample, a serum sample, a tissue sample, a urine sample, a saliva sample etc. The use of serum samples for analysis of the presence of the diagnostic marker molecules as described herein is preferred as well as the use of tissue samples. Tissue samples of biopsies taken from the prostate can be particularly preferred.

The term "marker" in the context of the present invention refers to any molecular entity or a combination of numerous molecular entities which can be used in the diagnosis of prostate cancer.

The term "marker molecule" in the context of the present invention refers to any polypeptide of a particular apparent molecular weight which is differentially present in a sample taken from patients being afflicted by ongoing prostate cancer development as compared to a comparable reference sample taken from control subjects, i.e. the human male subject with a negative diagnosis or undetectable prostate cancer development.

The terms "reference sample", "standard sample" or "control sample" all refer to samples which are taken from a human male subject with negative diagnosis prostate cancer or undetectable ongoing prostate cancer development. If such a control sample, standard sample, reference sample is said to be comparable to the sample that is taken from a human male subject being suspected to be afflicted by ongoing cancer development, this means that both samples, except for their expression profile have been derived and treated equally. Thus, the sample in both cases may e.g. be a tissue sample which has been further treated in a way to allow for detection of the diagnostic marker molecules as mentioned above.

The Marker molecules 1 to 29 as identified by the present invention are characterised by their molecular weight as indicated. While the absolute identities of these marker molecules are not yet known, such knowledge is not necessary to measure them in a patient sample because they are sufficiently characterised by e.g. their mass and additionally by affinity characteristics. In this context it is noted that molecular weight and binding properties are characteristic properties of polypeptides and thus of these marker molecules and are not limitations of means of detection or isolation. Furthermore, using the methods described herein or other methods known in the art, the absolute identity of such marker molecules can be determined. One example is described below.

In a preferred embodiment, the Marker molecules 1 to 29 in accordance with the present invention are not only characterised by their molecular weight, but also by their affinity towards an anion exchange resin of the Q10 type as available from Bio-Rad Laboratories Inc. (Hercules, CA, USA).

As mentioned above, the molecular weight masses of the marker molecules disclosed herein typically have an accuracy to be within ± 0.5 %, preferably within ± 0.3 %, and more within ± 0.2 % and even more preferably within ± 0.1 % of the disclosed molecular weight value. In a preferred embodiment Molecular markers 1 and 2 have an accuracy regarding their mass of 0.2% and 0.1 % respectively while the other Marker molecules have a mass accuracy of 0.5%.

The phrase "differentially expressed" or "differentially present" refers to differences in the quantity of marker molecules in accordance with the present invention in a sample taken from patients being afflicted by ongoing prostate cancer development as compared to a control sample being taken from a human male subject which has a negative diagnosis for prostate cancer or does not show any signs of ongoing prostate cancer development. Some marker molecules as described herein are present in elevated levels in samples of cancer patients compared to control samples from non-cancer patients. In contrast, other marker molecules as described herein are present at a decreased level in samples of cancer patients versus control samples from non-cancer patients.

A polypeptide such as those from which Marker molecules 1 to 29 in accordance with the present invention are derived, is differentially present within two samples if the amount of the polypeptide in one sample is significantly different from the amount of the polypeptide in the other sample. This difference may be statistically significant. For example, a polypeptide such as a marker molecule in accordance with the present invention is differentially present between two samples if it is present at least about 120 %, at least about 130 %, at least about 150%, at least about 180 %, at least about 200 %, at least about 300 %, at least about 500 %, at least about 700 %, at least about 900 % or at least about 1000 % greater than it is present in the other sample or if it is detectable in one sample and not detectable in the other. To determine whether a difference is statistically significant one can use available software such as e.g. the ProteinChip® of Ciphergen Biosystems, Inc. (Fremnont, CA, US) which compures p-values for each mass cluster. Similar software is commercially available from e.g. Bruker Daltonics Inc. and Waters Corporation. A value of p < 0.5 will be considered to be statistically significant.

The terms "polypeptide", "peptide" and "protein" are used interchangeably herein to refer to a polymer of amino acid residues. The terms include amino acid polymers in which one or more amino acid residue is an analogue or a mimetic of a corresponding naturally occurring amino acid as well as to naturally amino acid polymers. The terms also include modifications of polypeptides by e.g. the addition of carbohydrate residues to form glycoproteins or phosphate groups to form phosphoprteins. Other modifications include e.g. sumyolation or ubiquitination.

As has already been mentioned, the present invention not only relates to Marker molecules 1 to 29 as discussed above, but also to the use of these marker molecules in diagnosing prostate cancer in human male subjects. The present invention also relates to the aforementioned methods of diagnosing prostate cancer in human male subjects as well as to methods of aiding a prostate cancer diagnosis.

All these aspects which relate to making a decision whether a human male subject is likely to suffer from ongoing prostate cancer development basically use the same series of steps:

First, a sample is drawn from a human male subject. Subsequently these samples are prepared to allow for mass spectrometry analysis of protein expression patterns. Then, having performed the mass spectrometry analysis Marker molecules 1 to 16 are identified and the amounts of these marker molecules are compared with the amounts in a reference sample. Depending on the differential expression of the marker molecules, a decision as to whether the subject suffers from prostate cancer can then be made.

The type of samples and human subjects which may be analysed in the context of the present invention have been described above.

If desired, the sample can then be prepared to allow for better detection of the marker molecules. Typically, sample preparation can involve fractionation of the sample and collection of the fractions determined to contain the molecular markers. Methods of pre-fractionation include for example biochemical approaches such as salt precipitation, size exclusion chromatography, ion exchange chromatography, heparin chromatography, affinity chromatography, immunoaffinity chromatography, sequential extraction, gel electrophoresis and liquid chromatography. Sample preparation can, of course, also include washing, fixing, staining and/or lysis of cells.

In one embodiment, one may for example take cells from tissue of prostate patients, wash the cells and subsequently lyse the cells.

For washing the cells, one may use buffers as they are common for this purpose, such as PBS, pH 7.4. If desired, the cells can be fixed on a solid support by incubating cells with e.g. 70 % ethanol.

Further, the cells may be stained for visualisation by stainings such as Mayers's Hämatoxylin.

Subsequently, if the cells have been washed, fixed and/or stained, they can be lysed by e.g. ultrasound treatment, French press treatment or other treatments that are commonly used to induce cell lyses. One can then continue with the aforementioned separation technologies in order to obtain a pre-purified sample.

In one of the preferred embodiments of the present invention, one can pass a sample over an ion exchange matrix such as the aforementioned Q10-type matrix. Once the samples have been isolated, prepared and/or pre-fractionated, they may be analysed by mass spectrometry.

There are various forms of mass spectrometry based analysis with some of the more preferred variations thereof being shortly discussed in the following.

In MALDI-MS, a sample is mixed with a solution containing a matrix material and a drop of the liquid is placed on the surface of a probe. The matrix solution then e.g. co-crystallizes with the biological sample and the probe is inserted into the mass spectrometer and laser energy is then directed to the probe surface where it absorbs and ionizes the biological molecules without significantly fragmenting them. In other embodiments of MALDI-MS, the matrix may be first crystallized as a thin film with the biological sample being added later on or vice versa.

In the context of the present invention it can be preferred to not perform classical MALDI-MS analysis for detection of the marker molecules in accordance with the invention, but to rely on developments of MALDI-MS. These developments which include e.g. "surface enhanced laser desorbtion/ionization mass spectrometry (SELDI-MS) combine the above-mentioned principle of sample preparation and pre-fractionation with the principles of MALDI-MS.

In SELDI-MS, the probe surface is can e.g. be modified so that it forms an active participant in the sample preparation process. In one variant, this probe surface may therefore be derivatised with affinity matrices that provide an ion exchange characteristics to allow for pre-fractionation of the biological samples.

The principles of MALDI and SELDI are e.g. put forward in detail in e.g. US patent number 5,118,937, US 5,045,694, US 5,719,060 and US 2002/0060290 A1.

Further developments of the MALDI/SELDI technology are known in the art as surface-enhanced affinity capture (SEAC), surface-enhanced need disorption (SEND) or surface-enhanced photo label attachment and release (SEPAR). A person skilled in the art will consider all these mass spectrometry approaches for detecting the molecular markers in accordance with the present invention in a sample derived from a human male subject being suspected to be afflicted by ongoing prostate cancer development.

The SELDI technology has been commercialised by Ciphergen Biosystems, Inc (Fremnont, CA, US)., in the form of the so-called ProteinChip ^{®}Platform. The aforementioned Q10 surface can be obtained from manufacturers as mentioned above.

Once one has obtained the samples from human males subjects, prepared the samples, optionally has pre-fractionated them and subjected to a mass spectrometry analysis a so called M/Z-pattern is recorded wherein each peak depicts a certain M/Z-value. These M/Z-values are then typically converted by computer-based logarithms into a molecular weight. The transformation of time of flight data of mass spectrometers to M/Z values involves the applications of algorithms that are well known in the art.

If one has obtained such a mass spectrum by analysing a sample from a subject being suspected to suffer from ongoing prostate cancer development and control sample the molecular masses are typically evaluated.

The evaluation of the mass spectra data can include a normalisation of the data as well as the comparison with mass spectrum data that have been obtained for a comparable reference sample.

The observed peaks can be normalised in a process whereby the height of each peak relative to some reference is calculated. For example the reference can be background noise generated by instruments and chemicals (e.g. energy absorbing molecules) which are set at zero in the scale. Then the signal strength detected for each molecule can be displayed in the form of relative intensities in the scale desired. Alternatively, a standard (e.g. a tissue protein) may be admitted with the sample so that a peak from the standard can be used as a reference to calculate relative intensities of the signals observed for each molecule.

The normalisation of data thus typically aims to reduce the signal noise of a mass spectrum. The person skilled in the art is familiar with analysing peaks and background subtraction in order to eliminate background signal noise. For example, one may subtract the spectrum background and then normalise the spectra to total ion current. In a subsequent step, a decision as to whether a peak is considered as a peak or not may be achieved by setting a limit for the signal noise ratio (SNR). For example, in one embodiment of the present invention a peak in a mass spectrum may be considered to represent a signal of a molecule if the peak has a SNR of >3, preferably >5.

Further analysis generally involves the identification of peaks in the spectrum that represents signals from an analysed sample. Peak selection can, of course, be done by eye. However, there is also software available e.g. as part of Ciphergen's ProteinChip® Software that automates the detection of the peaks. A software which can be used for multivariate analysis of spectra is e.g. SIMCA-P version 11.0.0.0 as available from Umetrics AB (Umea, Sweden).

One of the parameter for the decision of whether a peak as such is to be considered or not will be whether peaks are within ± 0.3 M/Z. According to these criteria may be grouped together over spectra.

If molecules with defined molecular weights have been identified e.g. according to this procedure, the peak height of a distinct peak will be measured as being indicative of the amount which is present for the specific molecule. The amount determined for a specific peak in a sample derived from a subject being suspected to suffer from prostate cancer will then be compared with the amount of the same peak in a reference sample.

If the amount of any Marker molecule 1 to 29 is different from the reference sample, diagnosis as to the presence of prostate cancer can be made.

In general, software programs as mentioned above function by identifying signals having signal to noise ratio above a selected threshold and labelling the mass of the peak at the centroid of the peak signal. In a useful application many spectra are compared to identify identical peaks present in some selected percentage of the mass spectra. One may for example in a certain aspect cluster all peaks appearing in the varied spectra of different patients within a defined mass range and assign a mass (M/Z) to all the peaks that are near the midpoint of the mass (M/Z) cluster.

Once peak signals have been identified and normalised and the peak height has been translated into a relative amount of the detected molecular marker, the amount of this molecular marker can be compared to the amount being present for the same molecular marker in a reference sample as defined above.

In the context of the present invention it has been found that at least one marker molecule of Marker molecules 1 to 29 can be used to predict prostate cancer development in a human male subj ect.

This means, if at least one of Marker molecules 1 to 29 is detected in a sample taken from a human male subject which is suspected of suffering from prostate cancer development, this human subject will be likely afflicted by ongoing prostate cancer development by the above mentioned probabilities.

Of course, the reliability of this analysis will increase if more marker molecules are considered.

The person skilled in the art is aware that not all of Marker molecules 1 to 29 may be detected in a single experiment but that detection of (groups of) marker molecules may depend on the mass spectrometry settings.

Thus, marker molecules 1 to 16 will typically be detected in settings as they are used for high molecular weight markers. The group of marker molecules 1 to 16 is therefore also designated as high molecular weight group. These settings include higher laser intensity (e.g. 196 on a SELDI-MS PBS IIc) and optimization range at higher mass (e.g. 20-200 kDa) in order to ionize, desorb and detect the larger proteins. If one uses the aforementioned ProteinChip® software of Ciphergen, one may consider peaks in the 5000 to 150000 Da range with a SNR>3. Peaks within ±0.3% M/Z can then be grouped together over spectra into so-called clusters. Then, the mean mass can be computed, and the mean mass and the individual peak intensities can be transferred to SIMCA-P Version 11.0.0.0 for further analysis.

Marker molecules 17 to 29 will typically be detected in settings as they are used for low molecular weight markers. The group of marker molecules 17 to 29 is therefore also designated as low molecular weight group. These settings include lower laser intensity (e.g. 170 on a SELDI-MS PBS IIc) and optimization range at lower mass (e.g. 1-20 kDa) in order to ionize, desorb and detect the smaller proteins. If one uses the aforementioned ProteinChip® software of Ciphergen, one may consider peaks in the 600 to 30000 Da range with a SNR>3. Peaks within ±0.3% M/Z can then be grouped together over spectra into so-called clusters. Then, the mean mass can be computed, and the mean mass and the individual peak intensities can be transferred to SIMCA-P Version 11.0.0.0 for further analysis.

In one preferred embodiment of the invention, the above mentioned aspects (marker molecules, use of marker molecules, method of diagnosis etc.) only relate to the group of marker molecules 1 to 16.

In another preferred embodiment of the invention, the above mentioned aspects (marker molecules, use of marker molecules, method of diagnosis etc.) only relate to the group of marker molecules 17 to 29.

In one embodiment, the present invention relies on a single marker molecule of Marker molecules 1 to 29 for assessing whether a human male subject suffers from ongoing prostate cancer development.

In a preferred embodiment, the at least one marker molecule considered for diagnosis will be selected by the order of preference for Marker molecules 1 to 29:
Marker molecule 26;
Marker molecule 25;
Marker molecule 27;
Marker molecule 11;
Marker molecule 10;
Marker molecule 1;
Marker molecule 14;
Marker molecule 20;
Marker molecule 4;
Marker molecule 16;
Marker molecule 15;
Marker molecule 28;
Marker molecule 12;
Marker molecule 2;
Marker molecule 21;
Marker molecule 17;
Marker molecule 8;
Marker molecule 19;
Marker molecule 18;
Marker molecule 9;
Marker molecule 23;
Marker molecule 7;
Marker molecule 6;
Marker molecule 3;
Marker molecule 5;
Marker molecule 22;
Marker molecule 24;
Marker molecule 13. and
Marker molecule 29.

The above order of preference is based on the p-values calculated in Example 2 and Example 3. Thus, Marker molecule 26 (p -value of 5.5 * 10⁻⁹) is most preferred if used as the sole marker because it has the lowest p-value while Marker molecule 29 (p-value of 0,969297) is least preferred id used as a single marker. It is to be noted that p-values typically relate to a univariate analysis and that selectivity and specificity of prostate cancer diagnosis can be increased, if more than one marker is considered.

It is, of course, apparent to the person skilled in the art that the likelihood of a correct analysis will increase if more than one marker molecule of Marker molecules 1 to 29 is considered.

Thus, it can be preferred to consider at least two marker molecules of Marker molecules 1 to 29, at least 3, at least 4, at least 5, at least 6, at least 7, at least 8, at least 9, at least 10, at least 11, at least 12, at least 13, at least 14, at least 15 or at least 16 marker molecules of the group consisting of Marker molecules 1 to 29.

If numerous marker molecules are considered for a diagnosis, the order by which the marker molecules are considered may follow the above indicated order. Thus, if at least one Marker is used, one may use most preferably Marker molecule 26, them Marker molecule 25 etc.

If at least two Marker molecules are considered, one may rely most preferably on Marker molecules 26 and 27, then on Marker molecules 26 and 27, then on Marker molecules 25 and 27 etc.

In a preferred embodiment, a group of at least 4 marker molecules, preferably of at least 8 marker molecules, more preferably of at least 12 marker molecules, even more preferably of at least 16 marker molecules and most preferably of at least 20 marker molecules may be considered. It can be preferred that the aforementioned groups of at least 4, at least 8, at least 12, at least 16 and of at least 20 marker molecules comprise at least:
Marker molecule 26;
Marker molecule 25;
Marker molecule 27; and
Marker molecule 11;

It can also be preferred that the aforementioned groups of at least 8, at least 12, at least 16 and of at least 20 marker molecules comprise at least:
Marker molecule 26;
Marker molecule 25;
Marker molecule 27;
Marker molecule 11;
Marker molecule 10;
Marker molecule 1;
Marker molecule 14; and
Marker molecule 20.

In yet another embodiment, the aforementioned groups of at least 12, at least 16 and of at least 20 marker molecules comprise at least:
Marker molecule 26;
Marker molecule 25;
Marker molecule 27;
Marker molecule 11;
Marker molecule 10;
Marker molecule 1;
Marker molecule 14;
Marker molecule 20.
Marker molecule 4;
Marker molecule 16;
Marker molecule 15;and
Marker molecule 28.

In yet another embodiment, the aforementioned groups of at least 16 and of at least 20 marker molecules comprise at least:
Marker molecule 26;
Marker molecule 25;
Marker molecule 27;
Marker molecule 11;
Marker molecule 10;
Marker molecule 1;
Marker molecule 14;
Marker molecule 20.
Marker molecule 4;
Marker molecule 16;
Marker molecule 15;
Marker molecule 28.
Marker molecule 12;
Marker molecule 2;
Marker molecule 21; and
Marker molecule 17.

In yet another embodiment, the aforementioned group of at least 20 marker molecules comprises at least:
Marker molecule 26;
Marker molecule 25;
Marker molecule 27;
Marker molecule 11;
Marker molecule 10;
Marker molecule 1;
Marker molecule 14;
Marker molecule 20;
Marker molecule 4;
Marker molecule 16;
Marker molecule 15;
Marker molecule 28;
Marker molecule 12;
Marker molecule 2;
Marker molecule 21;
Marker molecule 17;
Marker molecule 8;
Marker molecule 19;
Marker molecule 18; and
Marker molecule 9.

In one of the most preferred embodiments, the diagnostic marker set will comprise either Marker molecules 1 to 16 or 17 to 29. By using either Marker molecules 1 to 16 or 17 to 29 it is possible to correctly diagnose at least about 95%, and preferably at least about 97% of the investigated subjects with prostate cancer.

In one aspect of the invention, one will thus preferably consider markers of the group consisting of Marker molecules 1 to 16.

Preferably, at least one marker molecule considered for diagnosis will be selected by the order of preference for Marker molecules 1 to 16:
Marker molecule 11;
Marker molecule 10;
Marker molecule 1;
Marker molecule 14;
Marker molecule 4;
Marker molecule 16;
Marker molecule 15;
Marker molecule 12;
Marker molecule 2;
Marker molecule 8;
Marker molecule 9;
Marker molecule 7;
Marker molecule 6;
Marker molecule 3;
Marker molecule 5; and
Marker molecule 13.

The above order of preference is based on the p-values calculated in Example 2. Thus, Marker molecule 11 (p -value of 3.23 * 10⁻⁷) is most preferred if used as the sole marker because it has the lowest p-value while Marker molecule 13 (p-value of 0,370844) is least preferred id used as a single marker. It is to be noted that p-values typically relate to a univariate analysis and that selectivity and specificity of prostate cancer diagnosis can be increased, if more than one marker is considered.

It is, of course, apparent to the person skilled in the art that the likelihood of a correct analysis will increase if more than one marker molecule of Marker molecules 1 to 16 is considered.

Thus, it can be preferred to consider at least two marker molecules of Marker molecules 1 to 29, at least 3, at least 4, at least 5, at least 6, at least 7, at least 8, at least 9, at least 10, at least 11, at least 12, at least 13, at least 14, at least 15 or preferably all 16 marker molecules of the group consisting of Marker molecules 1 to 16.

If numerous marker molecules are considered for a diagnosis, the order by which the marker molecules are considered may follow the above indicated order. Thus, if at least one Marker is used, one may use most preferably Marker molecule 11, then Marker molecule 10 etc.

If at least two Marker molecules are considered, one may rely most preferably on Marker molecules 11 and 10, then on Marker molecules 11 and 1, then on Marker molecules 10 and 1 etc.

In a preferred embodiment, a group of at least 4 marker molecules, preferably of at least 8 marker molecules, more preferably at least 12 marker molecules, and most preferably of all 16 marker molecules may be considered.

It can be preferred that the aforementioned groups of at least 4, at least 8, at least 12 and preferably of all 16 marker molecules comprise at least:
Marker molecule 11;
Marker molecule 10;
Marker molecule 1; and
Marker molecule 14.

If at least four markers are considered, the likelihood for predicting prostate cancer will be at least about 80% and preferably at least 85%.

It can also be preferred that the aforementioned groups of at least 8, at least 12 and preferably of all 16 marker molecules comprise at least:
Marker molecule 11;
Marker molecule 10;
Marker molecule 1;
Marker molecule 14;
Marker molecule 4;
Marker molecule 16;
Marker molecule 15; and
Marker molecule 12.

If at least eight markers are considered, the likelihood for predicting prostate cancer will be at least about 85% and preferably at least 90%.

In yet another embodiment, the aforementioned groups of at least 12 and preferably of all 16 marker molecules comprise at least:
Marker molecule 11;
Marker molecule 10;
Marker molecule 1;
Marker molecule 14;
Marker molecule 4;
Marker molecule 16;
Marker molecule 15;
Marker molecule 12;
Marker molecule 2;
Marker molecule 8;
Marker molecule 9; and
Marker molecule 7.

If at least twelve markers are considered, the likelihood for predicting prostate cancer will be at least about 90% and preferably at least about 95%.

In one of the most preferred embodiments, the diagnostic marker set will comprise all Marker molecules 1 to 16. If all these Marker molecules 1 to 16 can be detected to be differentially expressed in a sample which is derived from a human male subject that is suspected to be afflicted by ongoing prostate cancer development, this human male subject is likely to suffer from ongoing prostate cancer development by a likelihood of at least about 95% and preferably at least about 97%.

In another aspect of the invention, one will only consider markers of the group consisting of Marker molecules 17 to 29.

Preferably, at least one marker molecule considered for diagnosis will be selected by the order of preference for Marker molecules 17 to 29:
Marker molecule 26;
Marker molecule 25;
Marker molecule 27;
Marker molecule 20;
Marker molecule 28;
Marker molecule 21;
Marker molecule 17;
Marker molecule 19;
Marker molecule 18;
Marker molecule 23;
Marker molecule 22;
Marker molecule 24; and
Marker molecule 29.

The above order of preference is based on the p-values calculated in Example 3. Thus, Marker molecule 26 (p -value of 5.5 * 10⁻⁹) is most preferred if used as the sole marker because it has the lowest p-value while Marker molecule 29 (p-value of 0.969297) is least preferred id used as a single marker. It is to be noted that p-values typically relate to a univariate analysis and that selectivity and specificity of prostate cancer diagnosis can be increased, if more than one marker is considered.

It is, of course, apparent to the person skilled in the art that the likelihood of a correct analysis will increase if more than one marker molecule of Marker molecules 17 to 29 is considered.

Thus, it can be preferred to consider at least two marker molecules of Marker molecules 17 to 29, at least 3, at least 4, at least 5, at least 6, at least 7, at least 8, at least 9, at least 10, at least 11, at least 12 and preferably all 13 marker molecules of the group consisting of Marker molecules 17 to 29.

If numerous marker molecules are considered for a diagnosis, the order by which the marker molecules are considered may follow the above indicated order. Thus, if at least one Marker is used, one may use most preferably Marker molecule 26, then Marker molecule 25 etc.

If at least two Marker molecules are considered, one may rely most preferably on Marker molecules 26 and 25, then on Marker molecules 26 and 27, then on Marker molecules 25 and 27 etc.

In a preferred embodiment, a group of at least 4 marker molecules, preferably of at least 8 marker molecules, more preferably at least 12 marker molecules, and most preferably all 13 markers of Marker molecules 17 to 29 may be considered.

It can be preferred that the aforementioned groups of at least 4, at least 8, at least 12 and preferably of all 13 markers of Marker molecules 17 to 29 comprise at least:
Marker molecule 26;
Marker molecule 25;
Marker molecule 27; and
Marker molecule 20.

If at least four markers are considered, the likelihood for predicting prostate cancer will be at least about 80% and preferably at least about 85%.

It can also be preferred that the aforementioned groups of at least 8, at least 12 and preferably of all 13 markers of Marker molecules 17 to 29 comprise at least:
Marker molecule 26;
Marker molecule 25;
Marker molecule 27;
Marker molecule 20;
Marker molecule 28;
Marker molecule 21;
Marker molecule 17; and
Marker molecule 19.

If at least eight markers are considered, the likelihood for predicting prostate cancer will be at least about 85% and preferably at least about 90%.

In yet another embodiment, the aforementioned groups of at least 12 and preferably of all 13 markers of Marker molecules 17 to 29 comprise at least:
Marker molecule 26;
Marker molecule 25;
Marker molecule 27;
Marker molecule 20;
Marker molecule 28;
Marker molecule 21;
Marker molecule 17;
Marker molecule 19;
Marker molecule 18;
Marker molecule 23;
Marker molecule 22; and
Marker molecule 24.

If at least twelve markers are considered, the likelihood for predicting prostate cancer will be at least about 90% and preferably at least 95%.

In one of the most preferred embodiments, the diagnostic marker set will comprise all Marker molecules 17 to 29. If all these Marker molecules 17 to 29 can be detected to be differentially expressed in a sample which is derived from a human male subject that is suspected to be afflicted by ongoing prostate cancer development, this human male subject is likely to suffer from ongoing prostate cancer development by a likelihood of at least about 95%, preferably of at least about 97 %.

The person skilled in the art will be aware that p-values can be one parameter to choose marker combinations for increasing the likelihood of a correct prostate cancer diagnosis. However, the skilled person will also consider other aspects such as the quality of the peaks, i.e. whether peaks are clearly above the SNR, nicely center around the centroid etc. Of course, one can consider the quality of the peaks, the p-values and other factors.

As far as the markers of Marker molecules 1 to 16 are concerned one may in one embodiment e.g. consider a group comprising at least:
Marker molecule 10;
Marker molecule 1;
Marker molecule 12;
Marker molecule 7;
Marker molecule 13.

This group of will allow prostate cancer diagnosis with a likelihood of at least about 85%.

In another embodiment, one may consider a group comprising at least:
Marker molecule 10;
Marker molecule 1;
Marker molecule 14;
Marker molecule 4;
Marker molecule 15;
Marker molecule 12;
Marker molecule 9;
Marker molecule 7;
Marker molecule 6;
Marker molecule 3;
Marker molecule 5; and
Marker molecule 13.

This group of will allow prostate cancer diagnosis with a likelihood of at least about 90%.

As far as the markers of Marker molecules 17 to 29 are concerned, one may in one embodiment e.g. consider a group comprising at least:
Marker molecule 25;
Marker molecule 19;
Marker molecule 18;
Marker molecule 22; and
Marker molecule 24.

This group of will allow prostate cancer diagnosis with a likelihood of at least about 85%.

In another embodiment, one may consider a group comprising at least:
Marker molecule 25;
Marker molecule 20;
Marker molecule 21;
Marker molecule 19;
Marker molecule 18;
Marker molecule 22; and
Marker molecule 24.

This group of will allow prostate cancer diagnosis with a likelihood of at least about 90%.

In a particularly preferred embodiment, the present invention will rely on a tissue sample that is obtained by a biopsy of prostate tissue as an analysis of this tissue sample should allow the most reliable assessment of whether the aforementioned marker molecules being indicative of prostate cancer development are present or not. Another preferred embodiment relates to the use of blood and/or serum samples due to the ease by which these samples are obtainable.

The present invention provides for numerous advantages. Thus, it allows for diagnosing prostate cancer by a rather high likelihood while keeping the parameters to be considered and the amount of work to be conducted during diagnosis at a minimum level. Furthermore, the analysis of samples by mass spectrometry as well as the comparison with reference samples can easily be automated allowing for high throughput analysis. Further, a mass spectrometry pattern-based analysis of e.g. tissue is less operator-dependent and a somewhat more objective method than e.g. the palpation analysis as currently performed. Given that sample contents in the context of detecting the presence of Marker molecules 1 to 29 are separated according to mass, mass spectrometry profiling does not necessarily require the need for labelling. This can lead to a reduction of process complexity in comparison to immuno-histochemical methods.

If more than one Marker molecule is considered, the mass spectrometric proteomic pattern represents a combination of several individual molecules making the detection method more reliable in terms of sensitivity as well as specificity compared to e.g. diagnostic procedures which are currently based only one or very few antibody tests.

The invention will now be illustrated with respect to some of its preferred embodiments by describing specific examples. However, these examples are not to be construed as limiting the scope of the invention in any way.

### EXAMPLES

### Example 1 - sample preparation

In one example, approximately 6 µm thick cryo sections of tissue obtained from biopsies of tissue of 20 subjects which either showed histological abnormalities indicative of prostate cancer (i.e. which were obtained from tissues affected by tumor development) or showed no histological abnormalities (i.e. which were obtained from tissue not affected by tumor development) were manually microdissected, prepared and analysed according to the following.

Approximately 100000 cells were fixed in 70% ethanol for 30s, washed in aqua bidest for 10s, stained in Mayer's Hämatoxylin for 30s, washed in aqua bidest for 10s, stained blue in 0,1 % sodium hydrogen carbonate for 10s, and finally fixed in 70% ethanol for 30s.

Samples were added to 25 µl buffer containing 8M urea, 1% CHAPS in PBS and 0.1mM PMSF. The mixture was briefly vortexed followed by ultrasound treatment (Bendelin Sonorex RK255, 160/320W) at 5-10°C for 10min for cell lysis. The lysate was centrifuged at 16060g (13.000 rpm) for 5min (Pico Biofuge Heraeus), and the supernatant was transferred to a new eppendorf tube and diluted 1:10 in 50mM TRIS-HCl and TritonX100 0.1% pH9.

SELDI-MS protein arrays of Q10 type, i.e. strong anion exchange1, were pretreated 2x 5min with 50mM TRIS-HCl and TritonX100 0.1% pH9.

The diluted lysate was incubated on the protein arrays for 60min with shaking at 600rpm (Eppendorf Thermomixer Comfort) at room temperature (20-21°C). Subsequently, the diluted lysate was removed from the arrays.

The protein arrays were washed 2x 5min with 10mM TRIS-HCl pH9, and 1x briefly with 10mM HEPES pH7.

Finally, 2 x 0.8ul 100% SPA4 was added to each sample spot of the protein arrays.

The arrays were analysed in a SELDI-ToF-MS PBS IIc4 with settings optimized for the low-mass range (peptide range):

### Example 2 - Detecting High Molecular Weight Markers

The following parameters were adhered to:
- Set high mass to 200000 Daltons, optimized from 20000 Daltons to 200000 Daltons
- Set starting laser intensity to 196
- Set starting detector sensitivity to 7
- Focus by optimization center
- Set Mass Deflector to Auto
- Set data acquistion method to Seldi Quantitation
- Set Seldi acquisition parameters 19. delta to 9. transients per to 15 ending position to 79
- Set warming positions with 2 shots at intensity 202 and Don't include warming shots

After spectrum acquisition, spectra were analysed. Using Ciphergen ProteinChip Software Version 3.2.1.1216, the mass range 5000-120000Da was analyzed. Spectrum background was subtractedPeaks were manually selected in the same software, and grouped together over spectra in clusters within a ±0.3% M/Z intervall. Clusters with mean peak intensity <0.5 over spectra were excluded. For the remaining clusters, the mean mass was computed, and the mean mass and the individual peak intensities were transferred to SIMCA-P Version 11.0.0.0. Peak intensities were pareto scaled, i.e. multiplied with s^{-0.5}, where s denotes the standard deviation of the peak intensity. It was seen that peak patterns based on a subset of 16 peaks could accurately distinguish between normal and cancerous tissue. These peaks are shown in Table 1 with their p-values.

**Table 1:**

| Marker molecule (#) | Molecular weight (Da) | p-Value |
|---|---|---|
| 1 | 8092 | 1.12*10⁻⁶ |
| 2 | 8293 | 0.000682 |
| 3 | 10104 | 0.218068 |
| 4 | 105101 | 8.64*10⁻⁵ |
| 5 | 13383 | 0.244293 |
| 6 | 14998 | 0.084992 |
| 7 | 15193 | 0.064672 |
| 8 | 15481 | 0.001652 |
| 9 | 15755 | 0.01888 |
| 10 | 16714 | 8.36*10⁻⁷ |
| 11 | 16915 | 3.23*10⁻⁷ |
| 12 | 19851 | 0.000308 |
| 13 | 22209 | 0.370844 |
| 14 | 22719 | 1.49*10⁻⁵ |
| 15 | 32741 | 9.73*10⁻⁵ |
| 16 | 33622 | 9.36*10⁻⁵ |

### Example 3 - Detecting Low Molecular Weight Markers

The following parameters were adhered to:- Set high mass to 30000 Daltons, optimized from 1000 Daltons to 30000 Daltons
- Set starting laser intensity to 170
- Set starting detector sensitivity to 6
- Focus by optimization center
- Set Mass Deflector to 600 Daltons
- Set data acquistion method to Seldi Quantitation
- Set Seldi acquisition parameters 19. delta to 6. transients per to 10 ending position to 79
- Set warming positions with 2 shots at intensity 180 and Don't include warming shots

After spectrum acquisition, spectra were analysed. Using Ciphergen ProteinChip Software Version 3.2.1.1216, the mass range 600-30000Da was analyzed. Spectrum background was subtractedPeaks with SNR>3 were automatically detected, and peaks within ±0.3% M/Z were grouped together over spectra. Clusters with mean peak intensity <0.5 over spectra were excluded. For the remaining clusters, the mean mass was computed, and the mean mass and the individual peak intensities were transferred to SIMCA-P Version 11.0.0.0. Peak intensities were pareto scaled, i.e. multiplied with s^{-0.5}, where s denotes the standard deviation of the peak intensity. It was seen that a subset of 13 peaks could accurately distinguish between normal and cancerous tissue. These peaks are shown in Table 2 with their p-values.

**Table 2:**

| Marker molecule (#) | Molecular weight (Da) | p-Value |
|---|---|---|
| 17 | 2971 | 0.001447 |
| 18 | 4018 | 0.017926 |
| 19 | 7524 | 0.002597 |
| 20 | 8479 | 2.02*e-5 |
| 21 | 12176 | 0.001144 |
| 22 | 13497 | 0.294246 |
| 23 | 15326 | 0.026229 |
| 24 | 16469 | 0.340779 |
| 25 | 16783 | 3.33*e-8 |
| 26 | 16912 | 5.5*e-9 |
| 27 | 17163 | 3.76*e-7 |
| 28 | 19795 | 0.000156 |
| 29 | 22189 | 0.969297 |

## Claims

1. A diagnostic marker for detecting prostate cancer comprising at least one marker molecule selected from the group consisting of:
Marker molecule 1 having a molecular weight of about 8092 Da;
Marker molecule 2 having a molecular weight of about 8296 Da;
Marker molecule 3 having a molecular weight of about 10104 Da;
Marker molecule 4 having a molecular weight of about 10511 Da;
Marker molecule 5 having a molecular weight of about 13383 Da;
Marker molecule 6 having a molecular weight of about 14998 Da;
Marker molecule 7 having a molecular weight of about 15193 Da;
Marker molecule 8 having a molecular weight of about 15481 Da;
Marker molecule 9 having a molecular weight of about 15755 Da;
Marker molecule 10 having a molecular weight of about 16714 Da;
Marker molecule 11 having a molecular weight of about 16915 Da;
Marker molecule 12 having a molecular weight of about 19851 Da;
Marker molecule 13 having a molecular weight of about 22209 Da;
Marker molecule 14 having a molecular weight of about 22719 Da;
Marker molecule 15 having a molecular weight of about 32741 Da;
Marker molecule 16 having a molecular weight of about 33622 Da;
Marker molecule 17 having a molecular weight of about 2971 Da;
Marker molecule 18 having a molecular weight of about 4018 Da;
Marker molecule 19 having a molecular weight of about 7524 Da;
Marker molecule 20 having a molecular weight of about 8479 Da;
Marker molecule 21 having a molecular weight of about 12176 Da;
Marker molecule 22 having a molecular weight of about 13497 Da;
Marker molecule 23 having a molecular weight of about 15326 Da;
Marker molecule 24 having a molecular weight of about 16469 Da;
Marker molecule 25 having a molecular weight of about 16783 Da;
Marker molecule 26 having a molecular weight of about 16912 Da;
Marker molecule 27 having a molecular weight of about 17163 Da;
Marker molecule 28 having a molecular weight of about 19795 Da; and
Marker molecule 29 having a molecular weight of about 22189 Da.

2. A diagnostic marker according to claim 1,
wherein said at least one marker molecule is preferably:
Marker molecule 11 having a molecular weight of about 16915 Da; or
Marker molecule 26 having a molecular weight of about 16912 Da.

3. A diagnostic marker according to claim 1,
comprising a group of at least 4 of said marker molecules, preferably a group of at least 8 marker molecules, more preferably a group of at least 12 marker molecules and most preferably a group of 16 marker molecules wherein the marker molecules are selected from the group consisting of:
Marker molecule 1 having a molecular weight of about 8092 Da;
Marker molecule 2 having a molecular weight of about 8296 Da;
Marker molecule 3 having a molecular weight of about 10104 Da;
Marker molecule 4 having a molecular weight of about 10511 Da;
Marker molecule 5 having a molecular weight of about 13383 Da;
Marker molecule 6 having a molecular weight of about 14998 Da;
Marker molecule 7 having a molecular weight of about 15193 Da;
Marker molecule 8 having a molecular weight of about 15481 Da;
Marker molecule 9 having a molecular weight of about 15755 Da;
Marker molecule 10 having a molecular weight of about 16714 Da;
Marker molecule 11 having a molecular weight of about 16915 Da;
Marker molecule 12 having a molecular weight of about 19851 Da;
Marker molecule 13 having a molecular weight of about 22209 Da;
Marker molecule 14 having a molecular weight of about 22719 Da;
Marker molecule 15 having a molecular weight of about 32741 Da; and
Marker molecule 16 having a molecular weight of about 33622 Da.

4. A diagnostic marker according to claim 3,
wherein said groups comprise at least:
Marker molecule 11 having a molecular weight of about 16915 Da;
Marker molecule 10 having a molecular weight of about 16714 Da;
Marker molecule 1 having a molecular weight of about 8092 Da; and
Marker molecule 14 having a molecular weight of about 22719 Da.

5. A diagnostic marker according to claim 1,
comprising a group of at least 4 of said marker molecules, preferably a group of at least 8 marker molecules, more preferably a group of at least 12 marker molecules and most preferably a group of 13 marker molecules wherein the marker molecules are selected from the group consisting of:
Marker molecule 17 having a molecular weight of about 2971 Da;
Marker molecule 18 having a molecular weight of about 4018 Da;
Marker molecule 19 having a molecular weight of about 7524 Da;
Marker molecule 20 having a molecular weight of about 8479 Da;
Marker molecule 21 having a molecular weight of about 12176 Da;
Marker molecule 22 having a molecular weight of about 13497 Da;
Marker molecule 23 having a molecular weight of about 15326 Da;
Marker molecule 24 having a molecular weight of about 16469 Da;
Marker molecule 25 having a molecular weight of about 16783 Da;
Marker molecule 26 having a molecular weight of about 16912 Da;
Marker molecule 27 having a molecular weight of about 17163 Da;
Marker molecule 28 having a molecular weight of about 19795 Da; and
Marker molecule 29 having a molecular weight of about 22189 Da.

6. A diagnostic marker according to claim 5,
wherein said groups comprise at least:
Marker molecule 26 having a molecular weight of about 16912 Da;
Marker molecule 25 having a molecular weight of about 16783 Da;
Marker molecule 27 having a molecular weight of about 17163 Da; and
Marker molecule 20 having a molecular weight of about 8479 Da.

7. A diagnostic marker according to any of claims 1 to 6,
wherein the marker molecules are obtainable by analyzing a sample from a subject which is suspected to be afflicted by prostate cancer, using mass spectrometry, preferably by matrix assisted laser desorption ionization mass spectrometry or electron spray ionization mass spectrometry, and more preferably by surface enhanced laser desorption ionization mass spectrometry.

8. Use of at least one marker molecule selected from the group consisting of:
Marker molecule 1 having a molecular weight of about 8092 Da;
Marker molecule 2 having a molecular weight of about 8296 Da;
Marker molecule 3 having a molecular weight of about 10104 Da;
Marker molecule 4 having a molecular weight of about 10511 Da;
Marker molecule 5 having a molecular weight of about 13383 Da;
Marker molecule 6 having a molecular weight of about 14998 Da;
Marker molecule 7 having a molecular weight of about 15193 Da;
Marker molecule 8 having a molecular weight of about 15481 Da;
Marker molecule 9 having a molecular weight of about 15755 Da;
Marker molecule 10 having a molecular weight of about 16714 Da;
Marker molecule 11 having a molecular weight of about 16915 Da;
Marker molecule 12 having a molecular weight of about 19851 Da;
Marker molecule 13 having a molecular weight of about 22209 Da;
Marker molecule 14 having a molecular weight of about 22719 Da;
Marker molecule 15 having a molecular weight of about 32741 Da;
Marker molecule 16 having a molecular weight of about 33622 Da;
Marker molecule 17 having a molecular weight of about 2971 Da;
Marker molecule 18 having a molecular weight of about 4018 Da;
Marker molecule 19 having a molecular weight of about 7524 Da;
Marker molecule 20 having a molecular weight of about 8479 Da;
Marker molecule 21 having a molecular weight of about 12176 Da;
Marker molecule 22 having a molecular weight of about 13497 Da;
Marker molecule 23 having a molecular weight of about 15326 Da;
Marker molecule 24 having a molecular weight of about 16469 Da;
Marker molecule 25 having a molecular weight of about 16783 Da;
Marker molecule 26 having a molecular weight of about 16912 Da;
Marker molecule 27 having a molecular weight of about 17163 Da;
Marker molecule 28 having a molecular weight of about 19795 Da; and
Marker molecule 29 having a molecular weight of about 22189 Da.

9. Use according to claim 8,
wherein said at least one marker molecule is preferably:
Marker molecule 11 having a molecular weight of about 16915 Da; or
Marker molecule 26 having a molecular weight of about 16912 Da.

10. Use according to claim 8,
wherein a group of at least 4 marker molecules, preferably a group of at least 8 marker molecules, more preferably a group of at least 12 marker molecules and most preferably a group of 16 marker molecules is used wherein the marker molecules are selected from the group consisting of:
Marker molecule 1 having a molecular weight of about 8092 Da;
Marker molecule 2 having a molecular weight of about 8296 Da;
Marker molecule 3 having a molecular weight of about 10104 Da;
Marker molecule 4 having a molecular weight of about 10511 Da;
Marker molecule 5 having a molecular weight of about 13383 Da;
Marker molecule 6 having a molecular weight of about 14998 Da;
Marker molecule 7 having a molecular weight of about 15193 Da;
Marker molecule 8 having a molecular weight of about 15481 Da;
Marker molecule 9 having a molecular weight of about 15755 Da;
Marker molecule 10 having a molecular weight of about 16714 Da;
Marker molecule 11 having a molecular weight of about 16915 Da;
Marker molecule 12 having a molecular weight of about 19851 Da;
Marker molecule 13 having a molecular weight of about 22209 Da;
Marker molecule 14 having a molecular weight of about 22719 Da;
Marker molecule 15 having a molecular weight of about 32741 Da; and
Marker molecule 16 having a molecular weight of about 33622 Da.

11. Use according to claim 10,
wherein said groups comprise at least:
Marker molecule 11 having a molecular weight of about 16915 Da;
Marker molecule 10 having a molecular weight of about 16714 Da;
Marker molecule 1 having a molecular weight of about 8092 Da; and
Marker molecule 14 having a molecular weight of about 22719 Da.

12. Use according to claim 8,
wherein a group of at least 4 marker molecules, preferably a group of at least 8 marker molecules, more preferably a group of at least 12 marker molecules and most preferably a group of 13 marker molecules is used wherein the marker molecules are selected from the group consisting of:
Marker molecule 17 having a molecular weight of about 2971 Da;
Marker molecule 18 having a molecular weight of about 4018 Da;
Marker molecule 19 having a molecular weight of about 7524 Da;
Marker molecule 20 having a molecular weight of about 8479 Da;
Marker molecule 21 having a molecular weight of about 12176 Da;
Marker molecule 22 having a molecular weight of about 13497 Da;
Marker molecule 23 having a molecular weight of about 15326 Da;
Marker molecule 24 having a molecular weight of about 16469 Da;
Marker molecule 25 having a molecular weight of about 16783 Da;
Marker molecule 26 having a molecular weight of about 16912 Da;
Marker molecule 27 having a molecular weight of about 17163 Da;
Marker molecule 28 having a molecular weight of about 19795 Da; and
Marker molecule 29 having a molecular weight of about 22189 Da.

13. Use according to claim 12,
wherein said groups comprise at least:
Marker molecule 26 having a molecular weight of about 16912 Da;
Marker molecule 25 having a molecular weight of about 16783 Da;
Marker molecule 27 having a molecular weight of about 17163 Da; and
Marker molecule 20 having a molecular weight of about 8479 Da.

14. Method of aiding a prostate cancer diagnosis in a subject comprising the step of:
a) detecting at least one marker in a sample obtained from said subject wherein the marker is selected from the group consisting of:
Marker molecule 1 having a molecular weight of about 8092 Da;
Marker molecule 2 having a molecular weight of about 8296 Da;
Marker molecule 3 having a molecular weight of about 10104 Da;
Marker molecule 4 having a molecular weight of about 10511 Da;
Marker molecule 5 having a molecular weight of about 13383 Da;
Marker molecule 6 having a molecular weight of about 14998 Da;
Marker molecule 7 having a molecular weight of about 15193 Da;
Marker molecule 8 having a molecular weight of about 15481 Da;
Marker molecule 9 having a molecular weight of about 15755 Da;
Marker molecule 10 having a molecular weight of about 16714 Da;
Marker molecule 11 having a molecular weight of about 16915 Da;
Marker molecule 12 having a molecular weight of about 19851 Da;
Marker molecule 13 having a molecular weight of about 22209 Da;
Marker molecule 14 having a molecular weight of about 22719 Da;
Marker molecule 15 having a molecular weight of about 32741 Da;
Marker molecule 16 having a molecular weight of about 33622 Da;
Marker molecule 17 having a molecular weight of about 2971 Da;
Marker molecule 18 having a molecular weight of about 4018 Da;
Marker molecule 19 having a molecular weight of about 7524 Da;
Marker molecule 20 having a molecular weight of about 8479 Da;
Marker molecule 21 having a molecular weight of about 12176 Da;
Marker molecule 22 having a molecular weight of about 13497 Da;
Marker molecule 23 having a molecular weight of about 15326 Da;
Marker molecule 24 having a molecular weight of about 16469 Da;
Marker molecule 25 having a molecular weight of about 16783 Da;
Marker molecule 26 having a molecular weight of about 16912 Da;
Marker molecule 27 having a molecular weight of about 17163 Da;
Marker molecule 28 having a molecular weight of about 19795 Da; and
Marker molecule 29 having a molecular weight of about 22189 Da.

15. Method for diagnosing prostate cancer in a subject comprising the steps of:
a) detecting at least one marker in a sample obtained from said subject wherein the marker is selected from the group consisting of:
Marker molecule 1 having a molecular weight of about 8092 Da;
Marker molecule 2 having a molecular weight of about 8296 Da;
Marker molecule 3 having a molecular weight of about 10104 Da;
Marker molecule 4 having a molecular weight of about 10511 Da;
Marker molecule 5 having a molecular weight of about 13383 Da;
Marker molecule 6 having a molecular weight of about 14998 Da;
Marker molecule 7 having a molecular weight of about 15193 Da;
Marker molecule 8 having a molecular weight of about 15481 Da;
Marker molecule 9 having a molecular weight of about 15755 Da;
Marker molecule 10 having a molecular weight of about 16714 Da;
Marker molecule 11 having a molecular weight of about 16915 Da;
Marker molecule 12 having a molecular weight of about 19851 Da;
Marker molecule 13 having a molecular weight of about 22209 Da;
Marker molecule 14 having a molecular weight of about 22719 Da;
Marker molecule 15 having a molecular weight of about 32741 Da;
Marker molecule 16 having a molecular weight of about 33626 Da;
Marker molecule 17 having a molecular weight of about 2971 Da;
Marker molecule 18 having a molecular weight of about 4018 Da;
Marker molecule 19 having a molecular weight of about 7524 Da;
Marker molecule 20 having a molecular weight of about 8479 Da;
Marker molecule 21 having a molecular weight of about 12176 Da;
Marker molecule 22 having a molecular weight of about 13497 Da;
Marker molecule 23 having a molecular weight of about 15326 Da;
Marker molecule 24 having a molecular weight of about 16469 Da;
Marker molecule 25 having a molecular weight of about 16783 Da;
Marker molecule 26 having a molecular weight of about 16912 Da;
Marker molecule 27 having a molecular weight of about 17163 Da;
Marker molecule 28 having a molecular weight of about 19795 Da; and
Marker molecule 29 having a molecular weight of about 22189 Da.
b) comparing the amount of said marker with the amount of the same marker in a reference sample obtained from a subject which is known to not be affected by prostate cancer,
c) diagnosing prostate cancer if said at least one marker molecule is found to be differentially expressed compared to said standard sample.
